# FASCICULE DE BREVET EUROPEEN

(11) **EP 0 539 460 B1**
(45) Date de publication et mention de la délivrance du brevet: **05.10.1994**
(21) Numéro de dépôt: 91913376.9
(22) Date de dépôt: 15.07.1991
(51) Int. Cl.: C07D 498/18, A61K 37/02, A61K 31/42, C07K 5/06, C07B 63/00

(54) **SELS DERIVES DE LA DIALCOYLAMINOALCOYLSULFONYL-26 PRISTINAMYCINE II B**
VON DIALKYLAMINOALKYL-26-SULFONYLPRISTINAMYCIN IIB ABGELEITETE SALZE
SALTS DERIVED FROM DIALKYLAMINOALKYLSULPHONYL-26 PRISTINAMYCIN II B

(30) Priorité: 16.07.1990 FR 9009037
(43) Date de publication de la demande: 05.05.1993
(73) Titulaire: RHONE-POULENC RORER S.A., 92160 Antony (FR)
(72) Inventeur: BARRIERE, Jean-Claude, F-91300 Massy (FR); PARIS, Jean-Marc, F-77360 Vaires-sur-Marne (FR); RADISSON, Xavier, F-69006 Lyon (FR); CORBET, Jean-Pierre, F-69130 Ecully (FR)
(74) Mandataire: Savina, Jacques
(86) Numéro de dépôt international: FR9100582
(87) Numéro de publication internationale: WO9201694

(56) Documents cités:
- EP-A- 0 252 720
- EP-A- 0 365 213
- FR-A- 2 576 022

## Description

La présente invention concerne de nouveau sels de la (dialcoylamino-2 alcoyl)sulfonyl-26 pristinamycine II_{B}.

La (dialcoylamino-2 alcoyl)sulfonyl-26 pristinamycine II_{B} de formule générale : 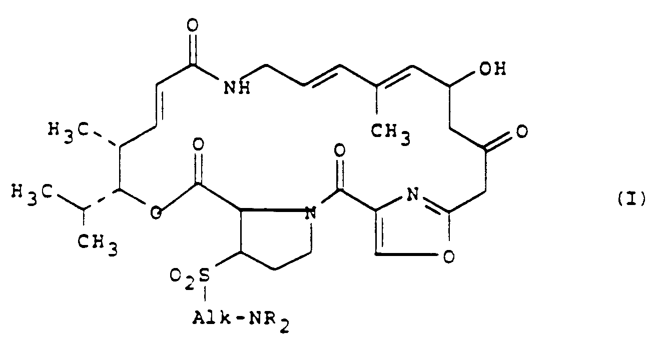
dans laquelle Alk représente un radical alcoylène droit ou ramifié et R représente des radicaux alcoyle droits ou ramifiés, ces radicaux contenant 1 à 10 atomes de carbone, sont des produits connus pour leur activité antibactérienne et leur action synergisante de l'activité antibactérienne de la pristinamycine I_{A} et de ses dérivés comme cela a été décrit dans le brevet européen 191 662.

Cependant les procédés d'oxydation qui conduisent à cette sulfone ne sont pas toujours totalement satisfaisants dans la mesure où ils conduisent à un produit souvent impur. Il est nécessaire d'effectuer des purifications ultérieures, notamment par chromatographie, pour parvenir à un produit de qualité suffisante.

De plus un faible choix de solvants sont utilisables pour le traitement de ces produits. Les sels préparés jusqu'à présent étaient solubles dans les solvants chlorés ou les cétones, solvants habituellement employés pour le traitement des sulfones de pristinamycine II_{B}. Aucun acide n'avait permi à ce jour de préparer des sels qui précipitent dans les solvants employés.

Dans la demande européenne EP 252 720 a été décrit un procédé d'oxydation conduisant aux (dialcoylamino-2 alcoyl) sulfonyl-26 pristinamycines II_{B} de formule générale (I).

Il a été maintenant trouvé que des sels dérivés de l'acide tartrique tels que le di-p.toluoyltartrate le di-t-butylacétyltartrate, le dibutyryltartrate et le di-i-valéryltartrate de la (dialcoylamino-2 alcoyl)sulfonyl-26 pristinamycine II_{B} sont des sels très insolubles dans certains solvants organiques et/ou qui précipitent facilement et de ce fait permettent de mettre en oeuvre la purification de cette sulfone avec de très bons résultats.

Les sels cités ci-dessus sont obtenus par salification par l'acide correspondant.

La réaction s'effectue dans les conditions habituellement utilisées qui n'altèrent pas le reste de la molécule. On opère dans un solvant chloré, notamment dans le chlorure de méthylène, le dichloroéthane, le chloroforme, le trichloréthylène ou le tétrachloroéthane, ou dans une cétone notamment la méthyléthylcétone, à une température comprise entre 10 et 25°C.

Les sels selon l'invention peuvent être retransformés par les méthodes habituelles pour libérer la base de départ ainsi purifiée.

Les nouveaux sels selon l'invention sont particulièrement intéressants du fait de leur insolubilité en permettant de surmonter les problèmes de purification qui existaient jusqu'à présent.

En outre les sels dérivés de l'acide tartrique, de la (dialcoylamino-2 alcoyl)sulfonyl-26 pristinamycine II_{B} présentent également des propriétés antibactériennes et des propriétés synergisantes de l'activité antibactérienne de la pristinamycine I_{A}, de la virginiamycine S et des dérivés solubles de la pristinamycine I_{A} et de la virginiamycine S, antérieurement décrits, notamment dans les brevets US 4 798 827 et US 4 618 599.

In vivo, ils synergisent l'activité antimicrobienne de la pristinamycine I_{A} sur les infections expérimentales de la souris à Staphylococcus aureus IP 8203 à une dose voisine de 75 mg/kg par voie orale (association 30/70).

Leur toxicité est supérieure à 750 mg/kg par voie sous-cutanée.

Les exemples suivants illustrent la préparation des produits selon l'invention.

### EXEMPLE 1

Dans un ballon monocol de 500 cm³, une solution de 10,861 g d'acide di-p-toluoyltartrique (26,05 mMole) dans 180 cm³ de dichlorométnane est refroidie à 15°C. On introduit en 22 minutes une solution de 24 g de (diéthylamino-2 éthyl) sulfonyl-26 pristinamycine II_{B} (26S) brute (titre en sulfone = 74,3 %) dans 180 cm³ de dichlorométhane, en agitant le mélange qui devient trouble après addition de 60 % de cette solution, et redevient complètement limpide en fin d'addition. Le mélange de ces deux solutions est légèrement exothermique. 10 minutes après la fin de l'addition, le sel commence à cristalliser. Après 3 heures, il est filtré, lavé par 3 fois 20 cm³ de dichlorométhane et séché sous pression réduite.

On obtient ainsi 23,37 g de sel titrant 100 %, soit un rendement réel de 82,6 %.

L'acide di-p-toluoyltartrique et la (diéthylamino-2 éthyl) sulfonyl-26 pristinamycine II_{B} (26S) manquantes sont intégralement contenus dans le filtrat : cette salification n'est donc pas dégradante.

### EXEMPLE 2

Une solution de 0,21 g d'acide dibutyryltartrique (L) (0,7237 mMole) dans 0,50 cm³ de méthyléthylcétone est additionnée goutte à goutte, en 4 minutes environ et sous agitation, à une solution de 1,047 g de (diéthylamino-2 éthyl)sulfonyl-26 pristinamycine II_{B} (26S) titrant 95,5 % (1,4474 mMole) dans 5 cm³ de méthyléthylcétone. La solution reste homogène jusqu'à la fin de l'addition. Le récipient contenant l'acide de départ est lavé 2 fois par 0,1 cm³ de méthyléthylcétone qui sont à leur tour additionnés au mélange. On obtient un précipité. Le mélange est agité 2 heures, puis filtré sur verre fritté (n° 4) et lavé par 0,5 cm³ puis 2 fois 1 cm³ de méthyléthylcétone. Après séchage sous pression réduite (2,7 kPa), on obtient 1,1233 g de dibutyryltartrate (L) de (diéthylamino-2 éthyl)sulfonyl-26 pristinamycine II_{B} sous forme d'un solide blanc-crème fondant à 150°C soit un rendement pondéral de 92,8 %.

Titre HPLC du sel ainsi obtenu : 97,5 %.

### EXEMPLE 3

En opérant comme à l'exemple 2, mais à partir de 15,6 g d'acide dibutyryltartrique (L) dans 150 cm³ de méthyléthylcétone et de 75 g de (diéthylamino-2 éthyl) sulfonyl-26 pristinamycine II_{B} (26S), dans 750 cm³ de méthyléthylcétone, on obtient, après 25 minutes d'agitation, un précipité. La suspension est placée à 0°C pendant 5 heures, puis le précipité est filtré sous atmosphère d'azote, rinçé par 100 cm³ puis 150 cm³ de méthyléthylcétone puis par 3 fois 200 cm³ de pentane puis séché sous pression réduite (13,5 kPa) à 30°C en présence de pentoxyde de phosphore. Le solide blanc obtenu (68 g) est ensuite battu sous atmosphère d'azote à l'aide d'une turbine à 5000 tours/mn pendant 15 minutes dans 700 cm³ de pentane, puis après filtration à nouveau 45 minutes à 6000 tours/mn dans 700 cm³ de pentane. Le solide est filtré sous azote, rinçé par 2 fois 100 cm³ de pentane puis séché en présence de pentoxyde de phosphore sous pression réduite (1,35 Pa) à 30°C. On obtient ainsi 59,9 g (88%) de dibutyryltartrate(L) de (diéthylamino-2 éthyl) sulfonyl-26 pristinamycine II_{B} (26S), sous forme d'un solide blanc fondant vers 150°C. Le titre HPLC est de 97,5%.

[α]_{D}²⁰ = -7,1°(c = 0,1 , H₂O)

### EXEMPLE 4

Une solution de 0,125 g d'acide di-t-butylacétyltartrique (L) (0,3619 mMole) dans 1,25 cm³ de méthyléthylcétone est additionnée goutte à goutte, sous agitation, à une solution de 0,5747 g de (diéthylamino-2 éthyl)sulfonyl-26 pristinamycine II_{B} (26S) titrant 87 % (0,7237 mMole) dans 3,75 cm³ de méthyléthylcétone. Un précipité se forme dès l'addition des quelques premières gouttes, jusqu'à obtention d'une masse épaisse en fin d'addition. Le mélange est agité 2 heures, puis filtré et lavé 3 fois par 1 cm³ de méthyléthylcétone. Après séchage sous pression réduite (0,13 kPa), on obtient 0,5785 g de di-t.butylacétyltartrate (L) de (diéthylamino-2 éthyl)sulfonyl-26 pristinamycine II_{B} (26S) sous forme d'un précipité blanc, soit un rendement pondéral de 92,64 %.

Titre du sel ainsi obtenu : 96,74 %.

### EXEMPLE 5

En opérant comme à l'exemple 4, mais à partir de 0,38 g d'acide di-t.butylacétyltartrique (L) dans 5 cm³ de méthyléthylcétone et de 1,5 g de (diéthylamino-2 éthyl)sulfonyl-26 pristinamycine II_{B} (26S) dans 10 cm³ de méthyléthylcétone, on obtient 1,2 g (67%) de di-t.butylacétyltartrate (L) de (diéthylamino-2 éthyl)sulfonyl-26 pristinamycine II_{B} (26S), sous forme d'un solide blanc fondant vers 153°C. Le titre HPLC est de 96,8%.

[α]_{D}²⁰ = -3,4° ± 0,8° (c = 0,51 , H₂O).

### EXEMPLE 6

Une solution de 0,23 g d'acide di-i-valéryltartrique (L) (0,7237 mMole) dans 0,5 cm³ de méthyléthylcétone est additionnée goutte à goutte, sous agitation, à une solution de 1,047 g de (diéthylamino-2 éthyl)sulfonyl-26 pristinamycine II_{B} titrant 95,5 % (1,4474 mMole) dans 5 cm³ de méthyléthylcétone. Un précipité se forme pendant l'addition de la solution. Le récipient contenant l'acide de départ est lavé par 0,2 cm³ de méthyléthylcétone qui sont à leur tour additionnés au mélange. Le mélange devenu épais est additionné de 1 cm³ de méthyléthylcétone. L'agitation est poursuivie pendant 2 heures, le mélange est filtré sur verre fritté (n° 4) et lavé par 0,5 cm³ puis 3 fois 1 cm³ de méthyléthylcétone. Après séchage sous pression réduite (0,13 kPa), on obtient 1,1245 g de di-i-valéryltartrate (L) de (diéthylamino-2 éthyl)sulfonyl-26 pristinamycine II_{B} (26S) sous forme d'un précipité blanc constitué de cristaux colorés dans une masse amorphe, soit un rendement pondéral de 91,4 %.

Titre du sel ainsi obtenu : 98,7 %.

### EXEMPLE 7

Dans un ballon monocol de 25 cm³ on ajoute sous agitation une solution de 0,35 g d'acide di-i.valéryltartrique (L) dans 5 cm³ de méthyléthylcétone à une solution 1,5 g de (diéthylamino-2 éthyl) sulfonyl-26 pristinamycine II_{B} (26S) dans 10 cm³ de méthyléthylcétone. Le précipité obtenu est filtré, lavé par 2 fois 3 cm³ de méthyléthyl cétone puis par 2 fois 20 cm³ de pentane. On obtient ainsi 1,66 g d'un solide blanc qui est recristallisé dans 20 cm³ de méthyléthylcétone bouillante. Les cristaux sont filtrés, rincés par 2 fois 3 cm³ de méthyléthylcétone puis par 3 fois 20 cm³ de pentane puis séchés sous pression réduite (2,7 kPa) à température ambiante. On obtient ainsi 1,23 g (66%) de di-i.valéryltartrate (L) de (diéthylamino-2 éthyl) sulfonyl-26 pristinamycine II_{B} (26S), sous forme d'un solide blanc fondant à 168 ± 5 °C. Le titre HPLC est de 96,7%.

[α]_{D}²⁰ = -6,7° ± 0,9° (c = 0,5 , H₂O).

Les acides diacyltartriques employés peuvent être préparés suivant la méthode decrite dans la demande européenne EP 007 834 et par Duhamel L. et Plaquevent J.C., Bull. Soc. Chim. France, II, 75-83 (1982).

Les sels selon l'invention peuvent être utilisés comme moyen de purification, comme illustré par l'exemple suivant :

### EXEMPLE D'UTILISATION

Dans cet essai, toute la procédure d'extraction est réalisée à 4°C.

2000 g de (diéthylamino-2 éthyl) sulfonyl-26 pristinamycine II_{B} (26S), di-p.toluoyltartrate sont ajoutés à un mélange de 38 litres d'eau et de 20 litres d'éther éthylique sous agitation. 500 cm³ d'acide sulfurique 1N sont ajoutés en 85 minutes à la suspension et le mélange agité 20 minutes (pH environ de 2). La phase aqueuse est décantée et extraite par 3 fois 10 litres d'éther éthylique puis par 3 fois 10 litres de pentane. La phase aqueuse est additionnée de 500 g de chlorure de sodium et de 10 litres de pentane et le mélange agité 10 minutes. La phase aqueuse est décantée, additionnée de 10 litres de pentane puis le mélange est agité. Une solution de 500 g de bicarbonate de potassium dans 2500 cm³ d'eau est ajoutée en 70 minutes. Le pH de la phase aqueuse est de 6,8 à la fin de l'addition. L'agitation est poursuivie 15 minutes, la phase aqueuse est décantée puis extraite au dichlorométhane (2 fois 2,5 litres). Les phases organiques sont réunies, lavées par 5 litres d'eau puis séchées sur 1,5 kg de sulfate de magnésium puis filtrées sur verre fritté. Le filtre est lavé par 2 fois 1 litre de dichlorométhane. Les phases organiques sont concentrées sous pression réduite (1,35 kPa) à 40°C pour donner un sirop jaune. 2 litres de pentane sont ajoutés à ce résidu et le mélange agité pendant 10 minutes. 1 litre de solvant est évaporé sous pression réduite (2,7kPa) à 30°C puis 4 autres litres de pentane sont ajoutés.La suspension est agitée une nuit à 4°C. Le solide est filtré sur verre fritté N 3, rinçé au pentane (2 fois 2 litres) et séché sous pression réduite (0,067 kPa) à 40°C pendant 54 heures pour donner 1126 g de (diéthylamino-2 éthyl) sulfonyl-26 pristinamycine II_{B} (26S) sous forme d'une poudre jaune clair. Le titre HPLC est de 100%.

La présente invention concerne également les médicaments constitués par les sels selon l'invention du produit de formule générale (I), à l'état pur ou sous forme d'une association avec tout diluant ou adjuvant compatible et pharmaceutiquement acceptable et/ou en association avec la pristinamycine I_{A}, la virginiamycine S ou un dérivé soluble de la pristinamycine I_{A} ou de la virginiamycine S défini notamment dans les brevets US 4 798 827 et US 4 618 599. Les médicaments selon l'invention peuvent être utilisés par voie orale, rectale ou topique.

Comme compositions pour administration orale peuvent être utilisés des comprimés, des pilules, des poudres ou des granulés. Dans ces compositions le produit actif éventuellement sous forme d'association, est mélangé à un ou plusieurs diluants ou adjuvants inertes, tels que saccharose, lactose ou amidon. Ces compositions peuvent également comprendre des substances autres que des diluants, par exemple un lubrifiant tel que le stéarate de magnésium.

Les compositions pour administration rectale sont les suppositoires ou les capsules rectales, qui contiennent outre le produit actif des excipients tels que le beurre de cacao, des glycérides semi-synthétiques ou des polyéthylèneglycols.

Les compositions pour administration topique peuvent être par exemple des crèmes, des pommades, des lotions ou des aérosols.

En thérapeutique humaine, le nouveau sel selon l'invention est particulièrement utile dans le traitement des infections d'origine bactériennes. Les doses dépendent de l'effet recherché et de la durée du traitement. Pour un adulte, elles sont généralement comprises entre 2000 et 4000 mg par jour.

D'une façon générale le médecin déterminera la posologie qu'il estime la plus appropriée en fonction de l'âge, du poids et de tous les autres facteurs propres au sujet à traiter.

L'exemple suivant illustre une composition selon l'invention.

### EXEMPLE

On prépare selon la technique habituelle des comprimés dosés à 250 mg de produit actif, ayant la composition suivante:

| | |
|---|---|
| - di-p.toluoyltartrate (L) de la (diéthylamino-2 éthyl)sulfonyl-26 pristinamycine II_{B} (26S) | 256,7 mg |
| - pristinamycine I_{A} | 75 mg |
| - excipient : amidon, silice hydratée, dextrine, gélatine stéarate de magnésium : qsp | 500 mg |

## Revendications (Revendications pour l'(les) Etat(s) contractant(s) suivant(s): AT, BE, CH, DE, DK, FR, GB, IT, LI, LU, NL, SE)

1. Sels de la (dialcoylamino-2 alcoyl)sulfonyl-26 pristinamycine II_{B} (26S) de formule générale : 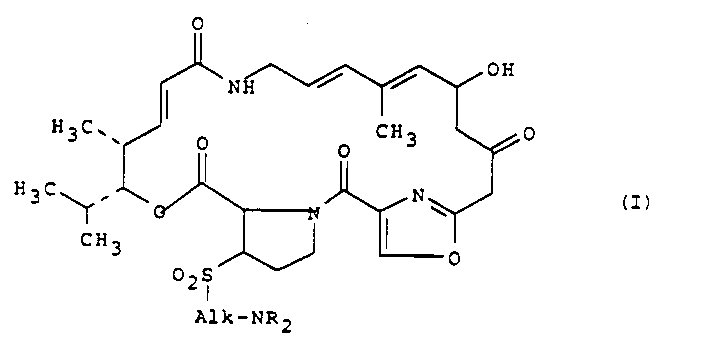 dans laquelle Alk représente un radical alcoylène droit ou ramifié et R représente des radicaux alcoyle droits ou ramifiés, ces radicaux contenant 1 à 10 atomes de carbone, caractérisé en ce qu'ils sont choisis parmi le di-p.toluoyltartrate , le di-t-butylacétyltartrate, le di-butyryltartrate et le di-i-valéryltartrate.

2. Composé selon la revendication 1, de formule: Le di-p-toluoyltartrate de (diéthylamino-2 éthyl) sulfonyl-26 pristinamycine II_{B} (26S).

3. Composé selon la revendication 1, de formule: Le di-t-butylacétyltartrate de (diéthylamino-2 éthyl sulfonyl-26 pristinamycine II_{B} (26S).

4. Composé selon la revendication 1, de formule: Le di-butyryltartrate de (diéthylamino-2 éthyl sulfonyl-26 pristinamycine II_{B} (26S).

5. Composé selon la revendication 1, de formule: Le di-i-valéryltartrate de (diéthylamino-2 éthyl sulfonyl-26 pristinamycine II_{B} (26S).

6. Composition pharmaceutique caractérisée en ce qu'elle comprend un sel selon la revendication 1, à l'état pur ou sous forme d'une association avec tout diluant ou adjuvant compatible et pharmaceutiquement acceptable et/ou avec la pristinamycine I_{A}, la virginiamycine S ou un dérivé soluble de la pristinamycine I_{A} ou de la virginiamycine S.

7. Utilisation d'un sel selon l'une des revendications 1 à 5 à titre de moyen de purification d'une (dialcoylamino-2 alcoyl) sulfonyl-26 pristinamycine II_{B} telle que définie dans la revendication 1.

## Revendications (Revendications pour l'(les) Etat(s) contractant(s) suivant(s): ES, GR)

1. Préparation de sels de la (dialcoylamino-2 alcoyl) sulfonyl-26 pristinamycine II_{B} de formule générale : 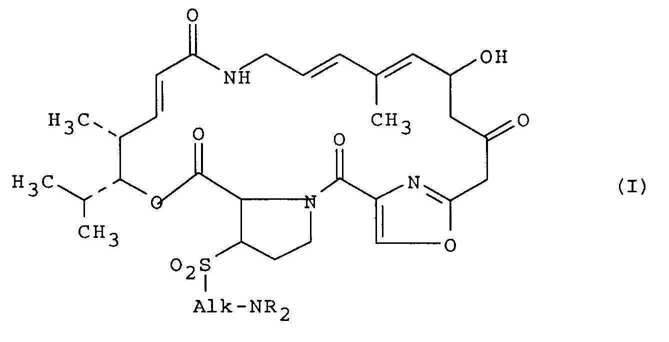 dans laquelle Alk représente un radical alcoylène droit ou ramifié et R représente des radicaux alcoyle droits ou ramifiés (ces radicaux contenant 1 à 10 atomes de carbone), choisis parmi le di-p. toluoyltartrate, le di-t.butylacétyltartrate, le di-butyryltartrate et le di-i.valéryltartrate, caractérisé en ce que l'on fait agir l'acide di-p-toluoyltartrique, l'acide di-t-butylacétyltartrique, l'acide di-butyrltartrique ou l'acide di-i-valéryltartrique sur la (dialcoylamino-2 alcoyl) sulfonyl-26 pristinamycine II_{B}.

2. Utilisation d'un sel préparé selon la revendication 1 à titre de moyen de purification d'une (dialcoylamino-2 alcoyl) sulfonyl-26 pristinamycine II_{B} telle que définie dans la revendication 1.

## Claims (Claims for the following Contracting State(s): AT, BE, CH, DE, DK, FR, GB, IT, LI, LU, NL, SE)

1. Salts of 26-[(2-dialkylaminoalkyl)sulphonyl]-pristinamycin II_{B} (26S) of general formula: 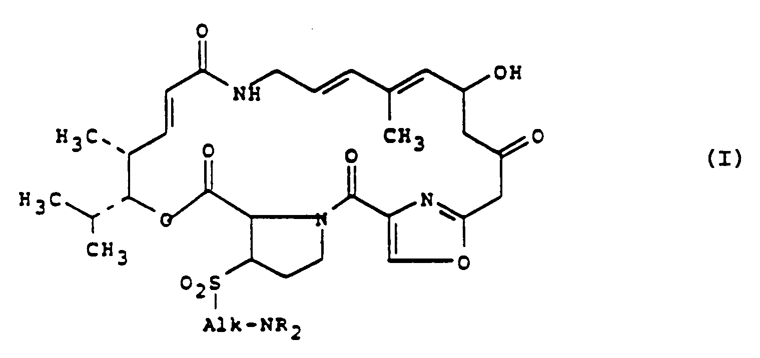 in which Alk represents a linear or branched alkylene radical and R represents linear or branched alkyl radicals, these radicals containing 1 to 10 carbon atoms, characterized in that they are chosen from among di-p-toluoyltartrate, di-t-butylacetyltartrate, di-butyryltartrate and di-i-valeryltartrate.

2. Compound according to claim 1, of formula: 26-[(2-diethylaminoethyl)sulphonyl]pristinamycin II_{B} (26S) di-p-toluoyltartrate.

3. Compound according to claim 1, of formula: 26-[(2-diethylaminoethyl)sulphonyl]pristinamycin II_{B} (26S) di-t-butylacetyltartrate.

4. Compound according to claim 1, of formula: 26-[(2-diethylaminoethyl)sulphonyl]pristinamycin II_{B} (26S) di-butyryltartrate.

5. Compound according to claim 1, of formula: 26-[(2-diethylaminoethyl)sulphonyl]pristinamycin II_{B} (26S) di-i-valeryltartrate.

6. Pharmaceutical composition characterized in that it comprises a salt according to claim 1, in the pure state or in the form of a combination with any compatible and pharmaceutically acceptable diluent or adjuvant and/or with pristinamycin I_{A}, virginiamycin S or a soluble derivative of pristinamycin I_{A} or of virginiamycin S.

7. Use of a salt according to one of claims 1 to 5 by way of purification means for a 26-[(2-dialkylaminoalkyl)sulphonyl]pristinamycin II_{B} such as defined in claim 1.

## Claims (Claims for the following Contracting State(s): ES, GR)

1. Preparation of salts of 26-[(2-dialkylaminoalkyl)sulphonyl]pristinamycin II_{B} of general formula: 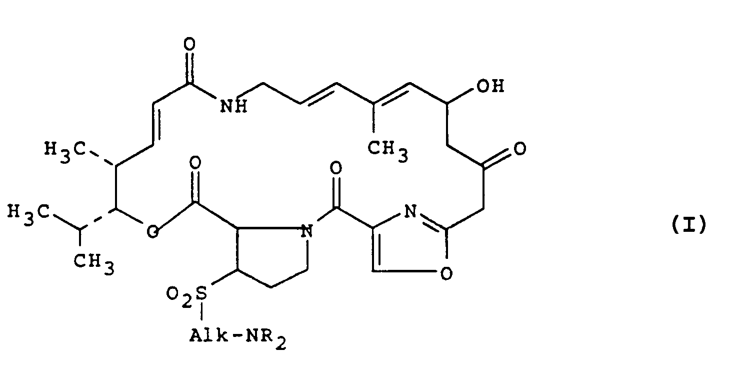 in which Alk represents a linear or branched alkylene radical and R represents linear or branched alkyl radicals (these radicals containing 1 to 10 carbon atoms) chosen from among di-p-toluoyltartrate, di-t-butylacetyltartrate, di-butyryltartrate and di-i-valeryltartrate, characterized in that di-p-toluoyltartaric acid, di-t-butylacetyltartaric acid, di-butyryltartaric acid or di-i-valeryltartaric acid is reacted with 26-[(2-dialkylaminoalkyl)sulphonyl]pristinamycin II_{B}.

2. Use of a salt prepared according to claim 1 by way of purification means for a 26-[(2-dialkylaminoalkyl)sulphonyl]pristinamycin II_{B} such as defined in claim 1.

## Patentansprüche (Patentansprüche für folgende(n) Vertragsstaat(en): AT, BE, CH, DE, DK, FR, GB, IT, LI, LU, NL, SE)

1. Salze von 2-Dialkylaminoalkyl-26-sulfonylpristinamycin II_{B} (26S) der allgemeinen Formel (I) 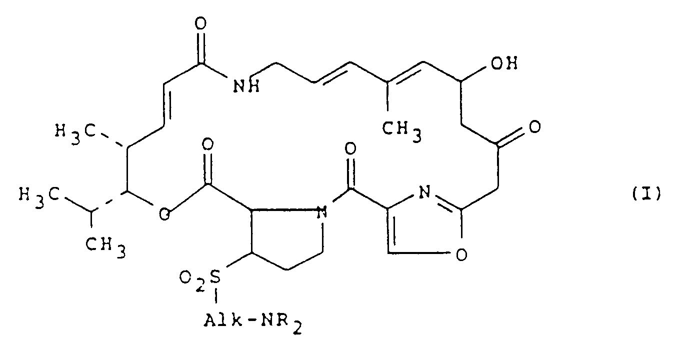 in der Alk einen geraden oder verzweigten Alkylenrest darstellt und R gerade oder verzweigte Alkylreste bedeutet, wobei diese Reste 1 bis 10 Kohlenstoffatome enthalten, dadurch gekennzeichnet, daß sie ausgewählt werden unter Di-para-toluyltartrat, Di-tert.-butylacetyltartrat, Di-butyryltartrat und Di-iso-valeryltartrat.

2. Verbindung nach Anspruch 1 der Formel Di-para-toluyltartrat von 2-Diethylaminoethyl-26-sulfonylpristinamycin II_{B} (26S).

3. Verbindung nach Anspruch 1 der Formel Di-tert.-butylacetyltartrat von 2-Diethylaminoethyl-26-sulfonylpristinamycin II_{B} (26S).

4. Verbindung nach Anspruch 1 der Formel Di-butyryltartrat von 2-Diethylaminoethyl-26-sulfonylpristinamycin II_{B} (26S).

5. Verbindung nach Anspruch 1 der Formel Di-iso-valeryltartrat von 2-Diethylaminoethyl-26-sulfonylpristinamycin II_{B} (26S).

6. Pharmazeutische Zusammensetzung, dadurch gekennzeichnet, daß sie ein Salz nach Anspruch 1 in reinem Zustand oder in Form einer Assoziation mit jedem kompatiblen und pharmazeutisch akzeptablen Verdünnungsmittel oder Zusatzstoff und/oder mit Pristinamycin I_{A}. Virginiamycin S oder einem löslichen Derivat von Pristinamycin I_{A} oder Virginiamycin S umfaßt.

7. Verwendung eines Salzes nach einem der Ansprüche 1 bis 5 als Mittel zur Reinigung eines 2-Dialkylaminoalkyl-26-sulfonylpristinamycins II_{B} wie in Anspruch 1 definiert.

## Patentansprüche (Patentansprüche für folgende(n) Vertragsstaat(en): ES, GR)

1. Herstellung von Salzen von 2-Dialkylaminoalkyl-26-sulfonylpristinamycin II_{B} der allgemeinen Formel (I) 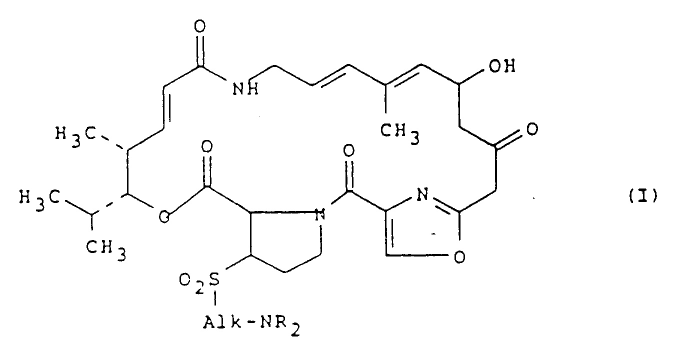 in der Alk einen geraden oder verzweigten Alkylenrest darstellt und R gerade oder verzweigte Alkylreste bedeutet, wobei diese Reste 1 bis 10 Kohlenstoffatome enthalten, ausgewählt unter Di-para-toluyltartrat, Di-tert.-butylacetyltartrat, Di-butyryltartrat und Di-iso-valeryltartrat, dadurch gekennzeichnet, daß man Di-para-toluylweinsäure, Di-tert.-butylacetylweinsäure, Di-butyrylweinsäure oder Di-iso-valerylweinsäure mit 2-Dialkylaminoalkyl-26-sulfonylpristinamycin II_{B} zur Reaktion bringt.

2. Verwendung eines nach Anspruch 1 hergestellten Salzes als Mittel zur Reinigung eines 2-Dialkylaminoalkyl-26-sulfonylpristinamycins II_{B} wie in Anspruch 1 definiert.
